# EUROPEAN PATENT SPECIFICATION

(11) **EP 2 278 025 B1**
(45) Date of publication and mention of the grant of the patent: **05.09.2012**
(21) Application number: 09725379.3
(22) Date of filing: 23.03.2009
(51) Int. Cl.: C12Q 1/48, C12N 9/12, G01N 33/574

(54) **METHOD FOR EVALUATION OF DEGREE OF MALIGNANCY OF TUMOR CELL**
VERFAHREN ZUR BEWERTUNG DES MALIGNITÄTSGRADS EINER TUMORZELLE
PROCÉDÉ POUR L'ÉVALUATION DU DEGRÉ DE MALIGNITÉ D'UNE CELLULE TUMORALE

(30) Priority: 28.03.2008 JP 2008087153
(43) Date of publication of application: 26.01.2011
(73) Proprietor: Sysmex Corporation, Kobe-shi, Hyogo 651-0073 (JP)
(72) Inventor: NOTOYA, Michitaka, Kobe-shi Hyogo 651-0073 (JP); SATO, Jun, Kobe-shi Hyogo 651-0073 (JP); ISHIHARA, Hideki, Kobe-shi Hyogo 651-0073 (JP)
(74) Representative: Paemen, Liesbet R.J.
(86) International application number: PCT/JP2009/055662
(87) International publication number: WO 2009/119502

(56) References cited:
- WO-A1-2007/009613
- JP-A- 2007 061 088
- JP-A- 2008 007 467
- JP-A- 2008 029 320
- US-A1- 2007 238 141
- HENNIPMAN A ET AL: "TYROSINE KINASE ACTIVITY IN BREAST CANCER BENIGN BREAST DISEASE AND NORMAL BREAST TISSUE", CANCER RESEARCH, vol. 49, no. 3, 1989, pages 516-521, XP002624655, ISSN: 0008-5472
- SCHRAAG B ET AL: "Standardization of an Enzyme-Linked Immunosorbent Assay for the Determination of Protein Tyrosine Kinase Activity", ANALYTICAL BIOCHEMISTRY, ACADEMIC PRESS INC, NEW YORK, vol. 211, no. 2, 1 June 1993 (1993-06-01), pages 233-239, XP024763659, ISSN: 0003-2697, DOI: DOI:10.1006/ABIO.1993.1262 [retrieved on 1993-06-01]
- VAN ERP H E ET AL: "PROTEIN TYROSINE KINASES IN HUMAN BRAIN AND GLIOMAS", JOURNAL OF NEUROCHEMISTRY, vol. 58, no. 2, 1992, pages 554-561, XP002624656, ISSN: 0022-3042
- OSKAM R ET AL: "PROTEIN PHOSPHORYLATION AND TYROSINE KINASE ACTIVITY IN MEDULLARY THYROID CARCINOMAS OF THE RAT", TUMOR BIOLOGY, KARGER, BASEL, CH, vol. 8, no. 4, 1 January 1987 (1987-01-01) , pages 186-202, XP001099145, ISSN: 1010-4283
- NOTOGAI R. ET AL.: 'Somaku Gabun Tyrosine Kinase Kassei Sokuteiho ni yoru Shinki Gan Shindanho' DAI 65 KAI NIHON GAN GAKKAI GAKUJUTSU SOKAI KIJI 28 August 2006, page 396

## Description

### TECHNICAL FIELD

The present invention relates to a method of evaluating the degree of malignancy of tumor cells. More specifically, the present invention relates to a method for evaluating the degree of malignancy of tumor cells by preparing, from a sample containing tumor cells, a cell membrane fraction containing receptor type tyrosine kinases (hereinafter referred to as "RTKs"), measuring an enzyme activity of RTKs in the cell membrane fraction in the presence and absence of an RTK activity inhibitor and comparing the thus obtained results.

### BACKGROUND ART

Kinases called RTKs are present in cell membranes. It is known that these kinases have important roles in cell differentiation and proliferation. RTKs include insulin-like growth factor receptors (IGFRs), platelet-derived growth factor receptors (PDGFRs), human epidermal growth factor receptors (HERs), vascular endothelial growth factor receptors (VEGFRs) and the like.

Previous studies have revealed that certain RTKs are over-expressed in certain tumor cells. For example, the expression level of a gene of HER2, one of the HERs, and of its protein is known to be increased in breast cancer. Then, it is considered that a possibility of recurrence of breast cancer may be estimated by examining the amount of HER2 in breast cancer patients. In addition, it has been reported that an antibody preparation Herceptin^{®} (generic name: trastuzumab) which is an inhibitor of HER2 has therapeutic effect against breast cancer.
Similarly, studies have been conducted on the relations between gene and/or protein expression levels of other RTKs and proliferation and metastasis of tumor cells. Studies have also been conducted on the effects of suppression of proliferation and metastasis of tumor cells by drugs targeting and inhibiting RTKs.

For example, Sirotnak et al. (Clinical Cancer Research, vol.6, p.4885-4892, 2000; Non-Patent Document 1) measured the expression level of HER1 gene (also referred to as EGFR), one of the HERs in pulmonary and prostate cancer cells, by RT-PCR (see Fig. 1 of Non-Patent Document 1). Then, they studied an impact of Iressa^{®} (generic name: gefitinib) which is an inhibitor of HER1 on the proliferation of the cancer cells (see Fig. 3 of Non-Patent Document 1). Sirotnak et al. reported that, based on the results, the proliferation of the tumor cell line (A-431) which had the highest expression level of HER1 was suppressed at the highest degree by Iressa^{®}. These results indicate that the higher the expression level of HER1 in tumor cells is, the higher the proliferation suppression effect Iressa^{®} has.

On the other hand, Ciardiello et al. (Clinical Cancer Research, vol.7, p.1459-1465, 2001; Non-Patent Document 2) indicate that the expression level of HER1 and IC50 by Iressa^{®} (a concentration (µM) at which a drug suppresses the proliferation of cells by 50%) are not always correlated. Specifically, it is indicated that the expression level of HER1 is high in MCF-7 ADR, OVCAR-3 and SW480 lines, while it is low in ZR-75-1 and KATO III lines (see Table 1 of Non-patent Document 2). On the other hand, IC50 values by Iressa^{®} are low in ZR-75-1 and KATO III lines as well as in MCF-7 ADR, OVCAR-3 and SW480 lines (see Table 2 of Non-patent Document 2). Namely, regardless of the expression level of HER1, the proliferation of these cell lines was suppressed by similar degree by Iressa^{®}.

The results of these studies show that the expression levels of RTK genes and /or proteins such as HER1 are not always correlated with the suppression effect in tumor cell proliferation by RTK inhibitors.
Hennipman et al. (Cancer Research, 49 (3): 516-521, 1989; Non-Patent Document 3) discloses a method for discriminating normal, benign and malignant breast tissue. The method involves bringing membrane fractions from the cells of the tissue into contact with a substrate having low specificity towards any particular type of RTK in order to measure the overall RTK activity of the fraction.
However, there is no disclosure or suggestion in the prior art with regard to the determination of cancer malignancies based on the measurement of the RTK activity both in the absence and in the presence of RTK inhibitors
Non-patent Document 1: Francis M. Sirotnak et al., "Efficacy of Cytotoxic Agents against Human Tumor Xenografts Is Markedly Enhanced By Coadministration of ZD1839 (Iressa), a Inhibitor of EGFR Tyrosine Kinase", Clinical Cancer Research, vol.6, p.4885-4892, 2000
Non-patent Document 2: Ciardiello et al., "Inhibition of Growth Factor Production and Angiogenesis in Human Cancer Cells by ZD1839 (Iressa), a Selective Epidermal Growth Factor Receptor Tyrosine Kinase Inhibitor", Clinical Cancer Research, vol.7, p.1459-1465, 2001
Non-patent Document 3: Hennipman et al., "Tyrosine kinase activity in breast cancer benign breast disease and normal breast tissue", Cancer Research, vol. 49, p.516-521, 1989.

### SUMMARY OF THE INVENTION

### Technical Problem

The inventors focused on the measurement of enzyme activity of RTKs (hereinafter referred to as "RTK activity"), instead of the measurements of gene and protein amount of RTKs, i.e. the expression level. Thus, the present invention aims to provide a method of evaluating the degree of malignancy of tumor cells based on the results of measurement of RTK activity.

### Means for Solving the Problems

The present invention provides a method for evaluating the degree of malignancy of tumor cells comprising the steps of:
(1) preparing a cell membrane fraction containing RTKs from a sample containing tumor cells;
(2) bringing the cell membrane fraction obtained in step (1) into contact with a first RTK activity inhibitor;
(3) bringing the cell membrane fraction having been contacted with the first RTK activity inhibitor in step (2) into contact with a substrate for at least two RTKs and measuring a phosphorylated substrate, so as to measure RTK activity in the presence of the first RTK activity inhibitor (first RTK activity);
(4) bringing the cell membrane fraction obtained in step (1) into contact with the same substrate as used in step (3) without having been contacted with the first RTK activity inhibitor and measuring a phosphorylated substrate, so as to measure RTK activity in the absence of the RTK activity inhibitor (control RTK activity);
(5) evaluating the degree of malignancy of the tumor cells in the presence of the first RTK activity inhibitor based on the first RTK activity and the control RTK activity.
wherein step (5) comprises the steps of:
(5a) calculating a relative value for RTK activity of the tumor cells based on the first and control RTK activities; and
(5b) comparing the relative value to a threshold value to evaluate the degree of malignancy of the tumor cells in the presence of the RTK inhibitor,
wherein either:
the relative value is a value obtained by dividing the first RTK activity by the control RTK activity, and if the relative value is lower than the threshold, the degree of malignancy of the tumor cells in the presence of the RTK inhibitor is evaluated to be low, or
the relative value is obtained by subtracting the first RTK activity from the control, RTK activity, and if the relative value is higher than the threshold, the degree of malignancy of the tumor cells in the presence of the RTK inhibitor is evaluated to be low.

In particular embodiments, step (1) of the method according to the present invention comprises the steps of:
(1 a) homogenizing the tumor cells in a buffer;
(1 b) obtaining an insoluble fraction from the homogenate obtained in step (1 a);
(1c) mixing to the insoluble fraction obtained in step (1b) with a solubilizing solution containing a surfactant; and
(1d) obtaining a soluble fraction from the mixture obtained in step (1c).

In further particular embodiments of the method according to the present invention, the substrate used in steps (3) and (4) is a substrate mixture in which multiple substrates having high specificities toward certain RTKs are combined, or a substrate having low specificity toward the type of RTKs.

In certain particular embodiments, the substrate having low specificity toward the type of RTKs comprises a peptide consisting of an amino acid sequence comprising a glutamic acid residue and a tyrosine residue.

In particular embodiments of the methods of the invention, the RTK activity inhibitor used in step (2) is an adenosine triphosphate (ATP) competitive inhibitor of RTKs.

In still further particular embodiments of the methods of the invention, the degree of malignancy of the tumor cells is proliferative ability and/or metastasizing ability of the tumor cells.

In some embodiments of the invention, the method further comprises the steps of:
(6) bringing the cell membrane fraction prepared in step (1) into contact with a second RTK activity inhibitor that is different from the first RTK activity inhibitor; and
(7) bringing the cell membrane having contacted with the second RTK activity inhibitor obtained in step (6) into contact with the same substrate as used in step (3) and measuring a phosphorylated substrate, so as to measure RTK activity in the presence of the second RTK activity inhibitor (second RTK activity), and
wherein step (5) further consists in evaluating the degree of malignancy of the tumor cells in the presence of the RTK inhibitors based on the second and control RTK activities.

In certain other particular embodiments, step (5) of the method of the invention further comprises the steps of:
calculating a second relative value for RTK activity of the tumor cells based on the second RTK activity and the control RTK activity; and
comparing the second relative value to respective corresponding threshold value to further evaluate the degree of malignancy of the tumor cells in the presence of the RTK inhibitors.

### Effect of the Invention

According to the present method, the degree of malignancy of tumor cells including proliferative ability and metastasizing ability in the presence of a RTK activity inhibitor can be easily evaluated by measuring RTK activity of samples containing tumor cells. According to the present method, the degree of malignancy of tumor of patients can be conveniently determined in short time by collecting tumor cells from the patients and measuring RTK activity of the cells. Thus, indices for the determination of effective therapeutic strategies for the patients (e.g. type of anticancer drugs to be used) can be easily obtained.

### BRIEF DESCRIPTION OF THE DRAWINGS

Fig. 1 shows cobweb charts presenting the results of Example 1.
Fig. 2 shows cobweb charts presenting the results of Example 1.
Fig. 3 shows graphs on proliferative ability of cells as the results of Comparative Example 1.
Fig. 4 shows the graph on the size of tumors as the results of Comparative Example 2.
Fig. 5 shows the graph on viability of cells as the results of Comparative Example 3.
Fig. 6 shows the graph on VEGF secretion ability of cells as the results of Comparative Example 4.
Fig. 7 shows the graph on invasive ability of cells as the results of Comparative Example 5.
Fig. 8 shows the graph on chemotactic ability of cells as the results of Comparative Example 6.

### BEST MODE FOR CARRYING OUT THE INVENTION

The present inventors focused on measuring RTK activities instead of measuring the expression levels of RTK genes and proteins. Further, the present inventors noted the reports revealing that:
- there are various types of RTKs;
- RTKs form homodimers through stimulation by ligands to transmit signals. However, RTKs also form multimers including heterodimers and tetramers, which are involved in the proliferation of tumor cells; and
- RTKs other than HER1 and HER2 are also involved in the cell proliferation and/or metastasis of tumor.
The present inventors have conceived from the above that in order to precisely obtain the information on the proliferation and metastasis of tumor cells, it is important to measure not only the activities of certain RTKs (e.g. HER1 and HER2) but also the activities of various kinds of RTKs (e.g. PDGFR, VEGFR etc.).

Thus, the present invention provides a method that makes it possible to measure the activities of multiple RTKs by measuring RTK activities in the presence or absence of a RTK activity inhibitor using a substrate which is specific for at least two RTKs.

As used herein, "the degree of malignancy of tumor cells" intends to include proliferative ability, metastasizing ability and the like of tumor cells. Preferably, the degree of malignancy of tumor cells is proliferative and/or metastasizing ability of tumor cells. The above "proliferative ability" includes the abilities of the cells that they can survive, that they are grown by increasing their volume and the like. The above "metastasizing ability" includes the ability of the tumor cells that they move from the original focus and stick to other tissues or organs, their VEGF (vascular endothelial growth factor) secretion ability, their chemotactic property (migration property: the ability that they move), their invasive ability (the ability that they invade tissues) and the like.

As used herein, RTKs are receptor type tyrosine kinases located on cell membranes and are also called trans-membrane tyrosine kinases. RTKs include insulin receptors (IRs), insulin-like growth factor receptors (IGFRs), platelet-derived growth factor receptors (PDGFRs), fibroblast growth factor receptors (FGFRs), human epidermal growth factor receptors (HERs), vascular endothelial growth factor receptors (VEGFRs) and the like.
These receptors are known to have several sub-families respectively, which are HER1, HER2, HER3, HER4 and the like for HER, for example.

According to the present method, a cell membrane fraction containing RTKs is prepared from a sample containing tumor cells.
The sample containing tumor cells may be derived from a biological specimen or from a tumor cell line culture. The biological specimen includes tissues (tumor tissues, organ tissues, lymph node tissues, blood and the like) obtained from biological body (patient) or celomic lavage fluid.

The preparation of the cell membrane fraction containing RTKs can be carried out with a method for separating cytoplasm from the sample containing tumor cells, namely, a method for separating from cytoplasm the cell membrane fraction to which RTKs are bound. Preferably, the preparation is carried out by a method comprising homogenizing tumor cells in an appropriate buffer (hereinafter referred to as "homogenizing reagent"), obtaining an insoluble fraction from the thus obtained homogenate, mixing the insoluble fraction with a solubilizing solution containing a surfactant and obtaining a soluble fraction from the mixture.
The homogenate obtained from the homogenization using the homogenizing reagent is separated into a soluble fraction (e.g. supernatant) and an insoluble fraction (e.g. pellet) by an appropriate method such as centrifugation. The soluble fraction contains cytoplasm-derived proteins and the like and the insoluble fraction contains cell membrane fragments which have various RTKs.
The mixture can be separated into a soluble fraction (e.g. supernatant) and an insoluble fraction (e.g. pellet) by an appropriate method such as centrifugation. The soluble fraction contains cell membranes having various RTKs which are solubilized with the surfactant (in micelles), and the insoluble fraction contains insoluble proteins, DNA and the like.

The cell membrane fraction containing RTKs preferably contains RTKs which are maintained in the cell membrane in such conformation that they can form multimers such as homodimers and heterodimers. The more preferred state is that such RTKs are maintained in the cell membrane and solubilized with the surfactant in micelles which are dispersed in a solution.
The preparation of the cell membrane fraction containing RTKs as above makes it possible to measure enzyme activities of various RTKs of tumor cells.

The homogenization of cells can be carried out by a method in which cell membrane can be fragmented. For example, such method includes pipetting, cell homogenization by freeze and thaw, mixing by voltex mixer, homogenization with a blender, pressurization with a pestle, sonication with a sonicator and the like.

The homogenizing reagent may be used for preventing denaturation of RTKs during the homogenization. The pH of the homogenizing reagent is not specifically limited so long as it is within a range in which RTKs can be recovered in a stable state without being denatured or deactivated. The pH of the homogenizing reagent is preferably 4.0 to 9.0, more preferably 4.5 to 8.5, and further more preferably 5.0 to 8.0.
The homogenizing reagent preferably contains a buffer. The buffer includes, for example, phosphate buffer, acetate buffer, citrate buffer, MOPS (3-morpholinopropanesulfonic acid), HEPES (2-[4-(2-hydroxyethyl)-1-piperazinyl]ethanesulfonic acid), Tris (Tris(hydroxymethyl)aminomethane), and TRICINE (N-[Tris(hydroxymethyl)methyl]glycine).

The surfactant contained in the solubilizing solution is not specifically limited so long as it can form micelles with fragmented cell membranes and does not degrade or denature RTKs contained in the cell membranes. The charged surfactants may possibly bind to RTKs to change their conformations, and therefore it is preferable to use nonionic surfactants that substantially do not bind to RTKs. Such nonionic surfactants include those having, as a fundamental structure, dodecyl ether, cetyl ether, stearyl ether, p-t-octylphenyl ether and the like. Specifically, nonionic surfactants include Nonidet^{®} P-40 (NP-40, Shell International Petroleum Company Limited), Triton-X^{®} (Union Carbide Chemical and Plastics Inc.), Tween^{®} (ICI Americas Inc.), Brij^{®} (ICI Americas Inc.) and Emulgen^{®} (Kao). The concentration of the surfactant in the solubilizing solution is preferably 0.05 to 5%, more preferably 0.1 to 3%, and further preferably 0.1 to 1%.

The solubilizing solution preferably contains a buffer similar to those used for the homogenizing reagent. The solubilizing solution preferably has similar pH as the homogenizing reagent.

The above homogenizing reagent and solubilizing solution may contain a protease inhibitor, a phosphatase inhibitor, a reagent for preventing the oxidization of SH group (hereinafter referred to as "SH group stabilizer") and the like.

The protease inhibitor can be used in order to prevent the degradation of RTKs by proteases contained in cells. The protease inhibitor includes, for example, metalloprotease inhibitors such as ethylenediaminetetraacetic acid (EDTA), glycoletherdiaminetetraacetic acid (EGTA); serine protease inhibitors such as phenylmethylsulfonyl fluoride (PMSF), trypsin inhibitor, chymotrypsin; cysteine protease inhibitors such as iodoacetamide, E-64. These protease inhibitors may be used alone or in combination.
A commercial product such as Protease Inhibitor Cocktail (Sigma) may be used, in which multiple protease inhibitors are mixed.

The phosphatase inhibitor can be used in order to prevent the decrease in RTK activities due to phosphatases contained in cells. The phosphatase inhibitor includes, for example, sodium orthovanadate (Na₃VO₄), sodium fluoride (NaF), okadaic acid and the like. These phosphatase inhibitors may be used alone or in combination.

The SH group stabilizer can be used in order to prevent deactivation of RTKs. SH groups of enzymes are liable to be oxidized to form stable disulfides. The formation of disulfides may result in deactivation of enzymes due to the change in the enzyme conformation. The SH group stabilizer for preventing such oxidation of SH groups includes reagents containing a SH group, which are, for example, dithiothreitol (DTT), 2-mercaptoethanol, glutathione, cysteine, homocysteine, coenzyme A, dihydrolipoic acid and the like.
The concentration of the SH group stabilizer in the homogenizing reagent and/or solubilizing solution is preferably 0.05 to 2 mM, more preferably 0.07 to 1.7 mM and further preferably 0.1 to 1.5 mM in case of DTT, for example. It is preferably 0.1 to 15 mM, more preferably 0.3 to 13 mM and further preferably 0.5 to 12 mM in case of 2-mercaptoethanol, for example.

The cell membrane fraction thus prepared is brought into contact with the RTK activity inhibitor. The RTK activity inhibitor is not specifically limited so long as it inhibits the activity of RTKs. The known RTK activity inhibitor includes the inhibitor which binds to an adenosine triphosphate (ATP)-binding site of RTKs (hereinafter referred to as "ATP competitive inhibitor" or "ATP competitive RTK activity inhibitor"), the inhibitor which binds to a substrate-binding site of RTKs, the inhibitor which binds to an extracellular domain (e.g. a ligand-binding site) of RTKs. Preferably, the RTK activity inhibitor is the ATP competitive inhibitor of RTKs.
The ATP competitive inhibitor of RTKs includes Iressa^{®} (AstraZeneca), Glivec^{®} (Novartis Pharma), Tarceva^{®} (OSI Pharmaceuticals, Inc.), PD153035 (Calbiochem), AG1478 (Calbiochem), 4557W (EGFR/ErbB-2 inhibitor) (Calbiochem), PDGF Receptor Tyrosine Kinase Inhibitor III (Calbiochem), VEGF Receptor Tyrosine Kinase Inhibitor III (Calbiochem) and the like.
The inhibitor which binds to a substrate-binding site of RTKs includes tyrphostin (Calbiochem) and the like.
The inhibitor which binds to an extracellular domain of RTKs includes Herceptin^{®} (Genentech), cetuximab (ImClone), pertuzumab (Genentech) and the like.

### The above contact is performed generally in a solution.

The concentration of the RTK activity inhibitor in the solution can be appropriately decided depending on the type of the inhibitor, the substrate concentration, a phosphate group donor (e.g. ATP) which is described herein below and the like. When, for example, ATP competitive inhibitor is used as the RTK activity inhibitor, the concentration of the inhibitor may be 0.1 to 10 folds of the concentration of the phosphate group donor (specifically ATP).

In the present method, the cell membrane fraction having contacted with the RTK activity inhibitor is brought into contact with a substrate for at least two RTKs and a phosphorylated substrate is measured to measure RTK activity in the presence of the RTK activity inhibitor (first RTK activity).
The above measurement is preferably carried out by mixing the cell membrane fraction, the substrate and the phosphate group donor and measuring the substrate phosphorylated by means of the activity of RTKs. Due to the reaction mediated by RTKs, a phosphate group of the phosphate group donor is incorporated into the substrate, making it possible to measure RTK activity by measuring the phosphorylated substrate.

The substrate for at least two RTKs is preferably a substrate having low specificity toward the type of RTKs (hereinafter referred to as "universal substrate") or a substrate mixture in which multiple substrates having high specificities toward certain RTKs are mixed. By measuring RTK activity with such substrate, activity of multiple RTKs in the sample can be measured.

The substrate having high specificity toward certain RTK(s), for example toward HER1, includes Grb2, myelin basic protein (MBP), histone H2B (HH2B), phospholipase C gamma and the like. The substrate having high specificity toward HER1 may be a commercially available substrate such as GST-EGFR substrate (Stratagene). GST-EGFR substrate is a fusion protein of glutathione-S-transferase (GST) and a substrate synthesized so as to be phosphorylated by the enzyme activity of HER1.

The above universal substrate is the substrate which has low specificity toward the type of RTKs, i.e. the substrate for multiple types of RTKs. Thus, the universal substrate is preferably a known synthetic peptide which is synthesized so as to have low specificity toward the type of RTKs. Such synthetic peptide specifically and preferably includes a peptide having an amino acid sequence comprising a glutamic acid residue and a tyrosine residue. Such synthetic peptide may be, for example, a commercially available synthetic peptide such as Poly(Glu4-Tyr) (biotin conjugate; UPSTATE). The synthetic peptide may further include the synthetic peptides described as substrates for tyrosine kinase in such references as Norio Sasaki et al., 1985, The Journal of Biological Chemistry, Vol.260, No.17, 9793-9804, Sergei Braun et al., 1984, The Journal of Biological Chemistry, Vol.259, No.4, 2051-2054, and M. Abdel-Ghany et al., 1990, Proceeding of The National Academy of Science, Vol.87, 7061-7065. The synthetic peptides disclosed in these references are consisting of amino acid sequences comprising a glutamic acid residue (Glu) and a tyrosine residue (Tyr) and synthesized so as to Tyr is phosphorylated by two or more tyrosine kinases.

The amino acid sequences of the above synthetic peptides specifically include, for example:
an amino acid sequence in which a sequence consisting of four Glu's and one Tyr is repeated two times or more (hereinafter referred to as "amino acid sequence a");
an amino acid sequence in which a sequence consisting of one Glu and one Tyr is repeated two times or more (hereinafter referred to as "amino acid sequence b");
an amino acid sequence in which a sequence consisting of six Glu's, one Tyr and three alanine residues (Ala) is repeated two times or more (hereinafter referred to as "amino acid sequence c");
an amino acid sequence in which a sequence consisting of one Glu, one Tyr and one Ala is repeated two times or more (hereinafter referred to as "amino acid sequence d");
an amino acid sequence in which a sequence consisting of two Glu's, one Tyr, six Ala's and five lysine residues (Lys) is repeated two times or more (hereinafter referred to as "amino acid sequence e"). The reference Tony Hunter, 1982, The Journal of Biological Chemistry, Vol.257, No.9, 4843-4848 reports that an acidic amino acid residue is important for the phosphorylation of Tyr by tyrosine kinase. Thus, the above substrate is preferably the amino acid sequences a and c which contain more Glu's, acidic amino acid residues.

The phosphate group donor includes, for example, adenosine triphosphate (ATP), adenosine 5'-O-(3-thiotriphosphate) (ATP- S), ³²P-labeled adenosine 5'-O-(3-triphosphate) ( -[³²P]-ATP), adenosine diphosphate (ADP), adenosine monophosphate (AMP) and the like.

The measurement of the phosphorylated substrate is preferably carried out by separating the phosphorylated substrate from other proteins and detecting the substrate.
In order to separate the phosphorylated substrate from other proteins, the substrate may have an affinity tag. By using the substrate having the affinity tag and a solid phase having a substance capable of binding to the affinity tag (hereinafter referred to as "binding substance"), it is made possible to easily separate the phosphorylated substrate from other proteins and collect it. Specifically, the phosphorylated substrate can be collected by collecting a complex of the substrate and the solid phase and dissociating the link between the affinity tag and the binding substance of the solid phase in the complex.

The affinity tag is not specifically limited so long as it has a corresponding binding substance and it does not prevent the binding between the substrate and RTKs or phsophorylation of the substrate. As the affinity tag, a polypeptide, hapten and the like may be used, for example. Specifically, GST, histidine, maltose binding protein, FLAG peptide (Sigma), Myc tag, hemagglutinin (HA) tag, Strep tag (IBA GmbH), biotin, avidin, streptavidin and the like may be used.

The substrate having the affinity tag may be the one obtained by chemically linking the affinity tag and the substrate. Alternatively, when the affinity tag is a polypeptide, the one obtained by introducing a vector, into a host, having a recombinant gene encoding a fusion protein of the affinity tag and the substrate and collecting the fusion protein produced by the host may also be used.

The binding substance corresponding to the affinity tag is not specifically limited so long as it can reversibly bind to the affinity tag. The binding substance includes, for example, glutathione, nickel, amylose, anti-FLAG antibody (Sigma), anti-Myc antibody, anti-HA antibody, Strep-Tactin (IBA GmbH) and the like.

The solid phase is not specifically limited so long as it is a support that can bind to the binging substance. The material for the solid phase includes, for example, polysaccharides, plastics, glass and the like. The shape of the solid phase includes, for example, beads, gel and the like. Specific examples of the solid phase include Sepharose beads, agarose beads, magnetic beads, glass beads, silicone gel and the like. These sold phases may be charged in columns for use.

The combinations of the affinity tag and the solid phase having the binding substance include the following examples.
When the affinity tag is GST, the solid phase used may be glutathione Sepharose beads (hereinafter referred to as "glutathione beads"). The measurement of RTK activity using this combination can be specifically carried out as follows. The cell membrane fraction having contacted with the RTK activity inhibitor is brought into contact with the substrate linked to GST. By adding glutathione beads thereto, glutathione beads to which the phosphorylated substrate has been bound are obtained. After collecting these glutathione beads, reduced glutathione is added to dissociate the link between GST and glutathione beads, and the substrate is collected.
Alternatively, the substrate linked to GST is first brought into contact with glutathione beads to obtain complexes of the substrate and the beads. Then, the cell membrane fraction having contacted with the RTK activity inhibitor is brought into contact with the complexes and is collected. Reduced glutathione is added thereto to dissociate the link between GST and glutathione beads, and the substrate is collected.

When the affinity tag is histidine, the solid phase having the binding substance can be nickel agarose beads, for example. The link between histidine and nickel can be dissociated with acid such as glycine-HCI or imidazole, for example.
When the affinity tag is maltose binding protein, the solid phase having the binding substance can be amylose magnetic beads, for example. The link between maltose binding protein and amylose can be dissociated with free amylose, for example.
When the affinity tag is FLAG peptide, the solid phase having the binding substance can be FLAG affinity gel (Sigma), for example. The link between FLAG peptide and FLAG affinity gel can be dissociated with acid such as glycine-HCI or 3xFLAG peptide (Sigma), for example.

When the affinity tag is Myc tag, the solid phase having the binding substance can be agarose beads bound to anti-Myc antibody.
When the affinity tag is HA tag, the solid phase having the binding substance can be agarose beads bound to anti-HA antibody.
The links between Myc tag and anti-Myc antibody and between HA tag and anti-HA antibody can be dissociated by denaturing proteins by adding acid or alkaline, for example. In these cases, the acid or alkaline to be used is preferably the one which allows the recovery of non-denatured protein. Specifically, the acid includes hydrochloric acid and alkaline includes sodium hydroxide.

When the affinity tag is Strep tag, the solid phase having the binding substance can be Strep-Tactin immobilized gel column (IBA GmbH), for example. The link between Strep tag and Strep-Tactin can be dissociated with desthiobiotin which reversibly react with streptavidin, for example.

After the contact of RTKs with the substrate, the enzyme reaction may be terminated by heating, cooling or addition of an enzyme inhibitor such as EDTA before collecting the phosphorylated substrate. By terminating the enzyme reaction, variation in measurement results from sample to sample can be prevented due to the progression of the enzyme reaction during collection of the substrate.

It is preferable to measure the phosphorylated substrate by attaching a labeling substance to the phosphorylated substrate separated from the solid phase and detecting the labeling substance. The labeling substance includes, but not limited to, for example, fluorescent substances, enzymes, radioisotopes and the like. The fluorescent substances include, for example, fluorescein, coumarin, eosin, phenanthroline, pyrene, rhodamine and the like. The enzymes include, for example, alkaline phosphatase, peroxidase and the like. The radioisotopes include, for example, ³²P, ³³P, ¹³¹I, ¹²⁵I, ³H, ¹⁴C, ³⁵S and the like.
When the labeling substance is the enzyme, the detection may be carried out by detecting a color derived from the reaction with the enzyme and its substrate. When the enzyme is an alkaline phosphatase, the substrate includes a mixture of nitrotetrazolium blue chloride (NBT) and 5-bromo-4-chloro-3-indoxyl phosphate (BCIP). When the enzyme is peroxidase, the substrate includes diaminobenzidine (DAB).

The linkage between the phosphorylated substrate and the labeling substance may be in a well-known manner. For example, the phosphorylated substrate can be linked to the labeling substance by using an antibody which has the labeling substance and can specifically bind to the phosphorylated substrate (hereinafter referred to as "phosphorylated substrate recognizing antibody").
The linkage between the phosphorylated substrate and the labeling substance may also be by means of the phosphorylated substrate recognizing antibody and an antibody which is capable of binding to the phosphorylated substrate recognizing antibody and has the labeling substance (hereinafter referred to as "secondary antibody"). In this case, the labeling substance can substantially be linked to the phosphorylated substrate via the phosphorylated substrate recognizing antibody and the secondary antibody.
The linkage between the phosphorylated substrate and the labeling substance may also be by means of the phosphorylated substrate recognizing antibody, the secondary antibody having biotin and avidin having the labeling substance. In this case, the labeling substance can substantially be linked to the phosphorylated substrate via the phosphorylated substrate recognizing antibody, the secondary antibody, biotin and avidin. The secondary antibody may have avidin and biotin may have the labeling substance.
The linkage between the phosphorylated substrate and the labeling substance may also be by means of the phosphorylated substrate recognizing antibody having biotin and avidin having the labeling substance, or of the phosphorylated substrate recognizing antibody having avidin and biotin having the labeling substance.
By using the labeling substance as above, the phosphorylated substrate can be detected by means of the signal generated by the labeling substance, making it possible to measure RTK activity.

The phosphorylated substrate recognizing antibody and secondary antibody may be antibodies obtained by a well-known method. Such methods include a method for obtaining antibodies from blood of an animal immunized with an antigen, a method for obtaining antibodies by gene recombination and the like. The antibodies may be polyclonal or monoclonal and may be a fragment of antibodies or a derivative thereof. These may be used in a mixture of two or more. The fragment and derivative of antibodies include Fab fragment, F(ab') fragment, F(ab)₂ fragment, sFv fragment (Blazaret al., 1997, Journal of Immunology, 159 : 5821-5833 and Bird et al., 1988, Science, 242 : 423-426) and the like. The antibody may be in any class of IgG, IgM and the like.

The detection method of the phosphorylated substrate may be appropriately selected according to the type of the labeling substance. For example, when the labeling substance is the fluorescent substance or enzyme, the phosphorylated substrate can be detected by Western blotting. The phosphorylated substrate can be detected by blotting to a membrane the phosphorylated substrate separated by electrophoresis such as SDS-PAGE, incubating the membrane in a buffer containing the phosphorylated substrate recognizing antibody to allow the binding between the antibody and the phosphorylated substrate, allowing the secondary antibody having the labeling substance to bind to the phosphorylated substrate recognizing antibody and detecting the labeling substance. When the phosphorylated substrate has the affinity tag, the phosphorylated substrate can be measured in a similar manner to the case of using Western blotting by separating the phosphorylated substrate using the affinity tag and carrying out slot blotting instead of Western blotting.

When the labeling substance is the fluorescent substance, the phosphorylated substrate can also be detected by placing a solution containing the phosphorylated substrate in a tube, adding the phosphorylated substrate recognizing antibody having the fluorescent substance in order to allow the binding of the antibody with the phosphorylated substance and measuring the fluorescent intensity.

When the labeling substance is the enzyme, the phosphorylated substrate can be detected by enzyme linked immunosorbent assay (ELISA). ELISA includes both direct adsorption and sandwich methods.
The direct adsorption method is a method comprising the direct adsorption of the phosphorylated substrate to the solid phase. The method may comprise, for example, adsorbing the phosphorylated substrate on the solid phase, allowing the binding between the phosphorylated substrate recognizing antibody having the enzyme and the phosphorylated substrate, developing color by using the substrate to the enzyme of the phosphorylated substrate recognizing antibody and detecting the generated color.
The sandwich method is a method comprising detecting the phosphorylated substrate by using two phosphorylated substrate recognizing antibodies which recognize two different sites of the phosphorylated substrate. The method may comprise, for example, allowing the phosphorylated substrate recognizing antibody (hereinafter referred to as "solid phase antibody") to bind to the solid phase, allowing the phosphorylated substrate to bind to the sold phase antibody, allowing the binding of the phosphorylated substrate recognizing antibody having the enzyme (hereinafter referred to as "labeling antibody") to the phosphorylated substrate and detecting color generated by the reaction between the enzyme of the labeling antibody and the substrate to the enzyme.

When the labeling substance is the radioisotope, the phosphorylated substrate can be detected by radioimmunoassay (RIA). Specifically, the phosphorylated substrate recognizing antibody having the radioisotope is linked to the phosphorylated substrate and radiation is measured by a scintillation counter and the like.

The step of measuring RTK activity in the absence of the RTK activity inhibitor (control RTK activity) in the present method is the same as the step of measuring the first RTK activity except that the cell membrane fraction is not brought into contact with the RTK activity inhibitor.

The present method comprises the step of evaluating the degree of malignancy of the tumor cells in the presence of the RTK activity inhibitor based on the first RTK activity and the control RTK activity.
The evaluating step is preferably carried out by calculating a relative value for RTK activity of tumor cells based on the first and control RTK activities and comparing the relative value to a threshold value (described hereinafter) to evaluate the degree of malignancy of the tumor cells in the presence of the RTK inhibitor.
The relative value is preferably a difference between the first RTK activity and the control RTK activity or a value obtained by dividing the first RTK activity by the control RTK activity (first RTK activity / control RTK activity), the latter being more preferred.

The threshold value is a value appropriately established according to the type of the RTK activity inhibitor or the type of tumor cells. The threshold value can be obtained by, for example, measuring first RTK activity and control RTK activity according to the method similar to the present method with tumor cells for which the degree of malignancy has been already clinically evaluated and a certain RTK activity inhibitor, and calculating the relative value.

In the evaluation step, when the relative value is obtained by subtracting the first RTK activity from the control RTK activity, the degree of malignancy of tumor cells in the presence of the RTK inhibitor can be evaluated to be low if the relative value is higher than the threshold. When the relative value is obtained by dividing the first RTK activity by the control RTK activity (first RTK activity / control RTK activity), the degree of malignancy of tumor cells in the presence of the RTK inhibitor can be evaluated to be low if the relative value is lower than the threshold.

The present method further comprises the steps of:
(a) bringing the cell membrane fraction containing RTKs prepared from the sample containing tumor cells into contact with a second RTK activity inhibitor that is different from the first RTK activity inhibitor; and
(b) bringing the cell membrane having contacted with the second RTK activity inhibitor obtained in step (a) into contact with the same substrate as used in the measurement for the first RTK activity and measuring a phosphorylated substrate, so as to measure RTK activity in the presence of the second RTK activity inhibitor (second RTK activity).
In the present method in which the second RTK activity is measured, the degree of malignancy of tumor cells in the presence of respective RTK inhibitors is evaluated based on the first, second and control RTK activities.
The combination of the first and second RTK activity inhibitors may be any combination among those listed as the RTK activity inhibitors. Multiple ATP competitive inhibitors of RTKs may be preferably selected.

The evaluation step comprises the steps of:
(a) calculating a first relative value for RTK activity of the tumor cells based on the first RTK activity and the control RTK activity;
(b) calculating a second relative value for RTK activity of the tumor cells based on the second RTK activity and the control RTK activity; and
(c) comparing the respective relative values calculated in steps (a) and (b) to respective corresponding threshold values to evaluate the degree of malignancy of the tumor cells in the presence of the RTK inhibitors.

The first and second relative values are the difference between the first or second RTK activity and the control RTK activity, or a value obtained by dividing the first or second RTK activity by the control RTK activity (first or second RTK activity / control RTK activity), the latter being more preferable.

The threshold value can be established in a similar manner as that described for the embodiment in which the first and control RTK activities are measured.

Accordingly, the use of multiple RTK activity inhibitors allows more precise and exhaustive evaluation of the degree of malignancy of tumor cells. The present method may give more accurate indices for the determination of effective therapeutic strategies for the patients (e.g. type of anticancer drugs to be used), possibly broadening the selection of clinical treatments.

### EXAMPLES

The present invention is now described in further details. However, it is not intended that these Examples are to limit the scope of the present invention.

### (Substrate for at least two RTKs)

The substrate for at least two RTKs used was Poly(Glu4-Tyr) (biotin conjugate, UPSTATE). Poly(Glu4-Tyr) is the substrate having low specificity toward the type of trans-membrane tyrosine kinases and specifically, is a fusion protein of biotin and a peptide having the amino acid sequence consisting of five sequences each of which consists of four glutamic acid residues and one tyrosine residue (hereinafter referred to as "Biotin-poly(Glu, Tyr) substrate").

### Example 1: Effect of ATP competitive inhibitors of RTK

In this Example, the cell membrane fraction containing RTKs was prepared from cultured tumor cells without purification of RTKs. The obtained cell membrane fraction was brought into contact with or without ATP competitive RTK inhibitors as RTK activity inhibitors, and RTK activities were measured with Biotin-poly(Glu, Tyr) substrate.

### (Preparation of cell membrane fraction)

Cultured cells (13 types: A-431, KF, MDA-MB-468, RMUG-L, BT-474, ES2, MDA-MB-453, SK-Br-3, SNG-S, K-562, MDA-MB-231, MCF7 and T47D) were respectively mixed with 1 ml of a homogenizing reagent (containing 20mM HEPES pH7.4, 0.2% protease inhibitors (PI), 10% glycerol, 200µM Na₃VO₄ and 50mM NaF), pressurized with a pestle to break cell membrane and cell homogenate was obtained. The obtained cell homogenate was centrifuged (20,000 x g, 20 min.), the supernatant was discarded and the pellet was collected. The collected pellet was added with a solubilizing solution (containing 20mM HEPES pH7.4, 1% NP40, 0.2% PI, 10% glycerol, 200µM Na₃VO₄ and 50mM NaF), mixed, pressurized with a pestle to solubilize cell membrane and centrifuged (20,000 x g, 20 min.). The supernatant was then collected as the cell membrane fraction, adjusted its total protein amount to 40µg/ml and used for the following experiments.

A-431 is a cell line derived from squamous cell carcinoma, and the cell membrane fraction prepared from this cell line is called as the cell membrane fraction A-431.
KF is a cell line derived from ovarian carcinoma, and the cell membrane fraction prepared from this cell line is called as the cell membrane fraction KF.
MDA-MB-468 is a cell line derived from breast carcinoma, and the cell membrane fraction prepared from this cell line is called as the cell membrane fraction MDA-MB-468.
RMUG-L is a cell line derived from ovarian carcinoma, and the cell membrane fraction prepared from this cell line is called as the cell membrane fraction RMUG-L.
BT-474 is a cell line derived from breast carcinoma, and the cell membrane fraction prepared from this cell line is called as the cell membrane fraction BT-474.
ES2 is a cell line derived from ovarian carcinoma, and the cell membrane fraction prepared from this cell line is called as the cell membrane fraction ES2.
MDA-MB-453 is a cell line derived from breast carcinoma, and the cell membrane fraction prepared from this cell line is called as the cell membrane fraction MDA-MB-453.
SK-Br-3 is a cell line derived from breast carcinoma, and the cell membrane fraction prepared from this cell line is called as the cell membrane fraction SK-Br-3.
SNG-S is a cell line derived from endometrial carcinoma, and the cell membrane fraction prepared from this cell line is called as the cell membrane fraction SNG-S.
K-562 is a cell line derived from leukemia, and the cell membrane fraction prepared from this cell line is called as the cell membrane fraction K-562.
MDA-MB-231 is a cell line derived from breast carcinoma, and the cell membrane fraction prepared from this cell line is called as the cell membrane fraction MDA-MB-231.
MCF7 is a cell line derived from breast carcinoma, and the cell membrane fraction prepared from this cell line is called as the cell membrane fraction MCF7.
T47D is a cell line derived from breast carcinoma, and the cell membrane fraction prepared from this cell line is called as the cell membrane fraction T47D.

### (Binding of Biotin-poly(Glu, Tyr) substrate to ELISA plate)

The ELISA plate used was the avidin coated plate (NeutrAvidin HBC White 96-Well Plates With SuperBlock Blocking Buffer; PIERCE). Each well of the plate was washed three times with TBS - T (containing 25mM Tris, 150mM NaCl and 0.05% Tween-20). A substrate solution 1 (50µl; TBS containing 1µg/ml Biotin-poly(Glu, Tyr) substrate) was added to each well and the plate was incubated with gentle shaking at 25°C for 1.5 hours. After the incubation, each well was washed three times with TBS-T. Thus, Biotin-poly(Glu, Tyr) substrate was bound to the surface of each well of the ELISA plate. This ELISA plate was used for the following enzyme reaction.

### (Contact of RTKs with ATP competitive inhibitors)

PD153035 (HER1 inhibitor; Calbiochem), AG1478 (HER1 inhibitor; Calbiochem), 4557W (HER1/HER2 inhibitor; Calbiochem), PDGF Receptor Tyrosine Kinase Inhibitor III (hereinafter referred to as "PDGFR inhibitor"; Calbiochem) and VEGF Receptor Tyrosine Kinase Inhibitor III (hereinafter referred to as "VEGFR inhibitor; Calbiochem), all of which were ATP competitive RTK activity inhibitors, were used. Src inhibitor (Calbiochem) was also used, which was the ATP competitive inhibitor of non-receptor tyrosine kinase, Src. The chemical formulae of these inhibitors are as follows.

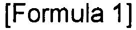

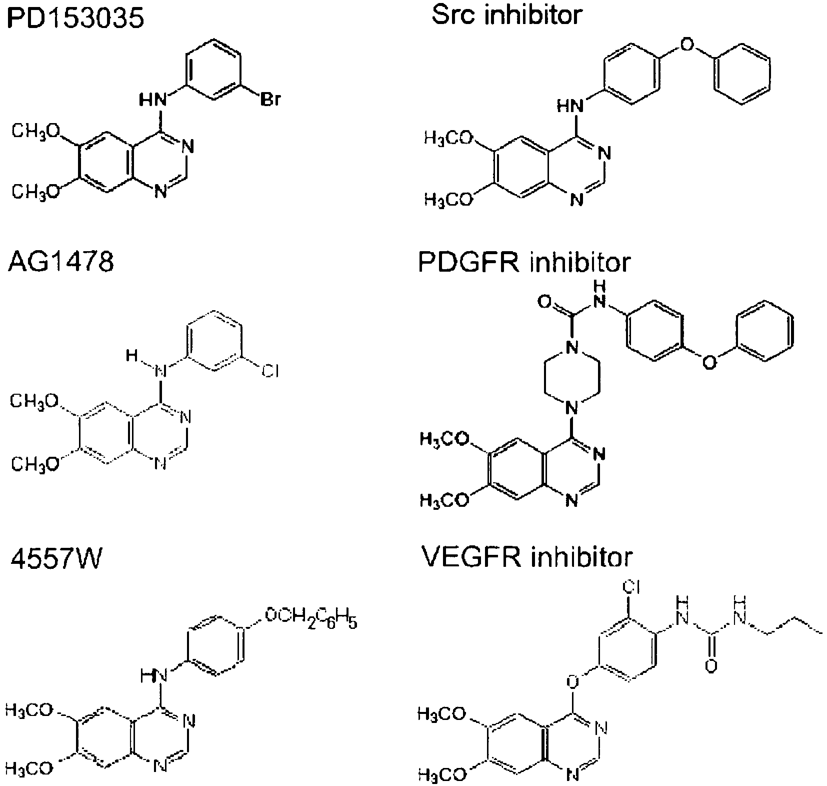

The cell membrane fractions (25µl each) of the above tumor cells were placed in seven tubes, respectively. To six tubes were added 25µl of a treatment solution (20mM HEPES pH7.4, 20mM MnCl₂, 2mM DTT, 1 % NP40, 10% glycerol, 200µM Na₃VO₄, 50mM NaF, 100µM ATP, 400µM PPI (BIOMOL)) containing 100µM PD153035, 25µl of treatment solution containing 100µM AG1478, 25µl of the treatment solution containing 100µM 4557W, 25µl of the treatment solution containing 100µM Src inhibitor, 25µl of the treatment solution containing 100µM PDGFR inhibitor and 25µl of the treatment solution containing 100µM VEGFR inhibitor, respectively, to contact the cell membrane fractions with RTK activity inhibitors. To the remained tube was added with 25µl of the treatment solution without inhibitors. The thus obtained solutions are called as reaction solutions. In order to suppress the enzyme activity of RTKs, the above procedures were carried out at below 4°C.

### (Measurement of RTK activity)

Each reaction solution (50µl) was distributed to each well of the ELISA plate prepared as above, and the plate was incubated at 25°C for about 30 minutes to bring the cell membrane fractions having contacted or not with RTK activity inhibitors into contact with the substrate. After the incubation, each well was added with 100µl of a reaction termination solution (TBS-T containing 5 mM EDTA) and washed three times with TBS-T. Each well was then washed with 300µl of StartingBlock T20 (TBS) Blocking Buffer (PIERCE). After washing, each well was added with 100µl of HRP-labeled primary antibody (p-Tyr (PY20), sc-508 HRP (SANTA Cruz Biotechnology), an antibody recognizing a phosphorylated tyrosine residue) diluted to 1000-fold with StartingBlock T20 (TBS) Blocking Buffer, and the plate was incubated with gentle shaking at 25°C for about 1.5 hours. After the incubation, wells were washed for five times with TBS-T. After washing, each well was added with 150µl of chemiluminescent substrate (SuperSignal ELISA PICO (PIERCE)), and after 5 min., luminescence was measured with a microplate reader GENios (TECAN) to measure RTK activities.

### (Results)

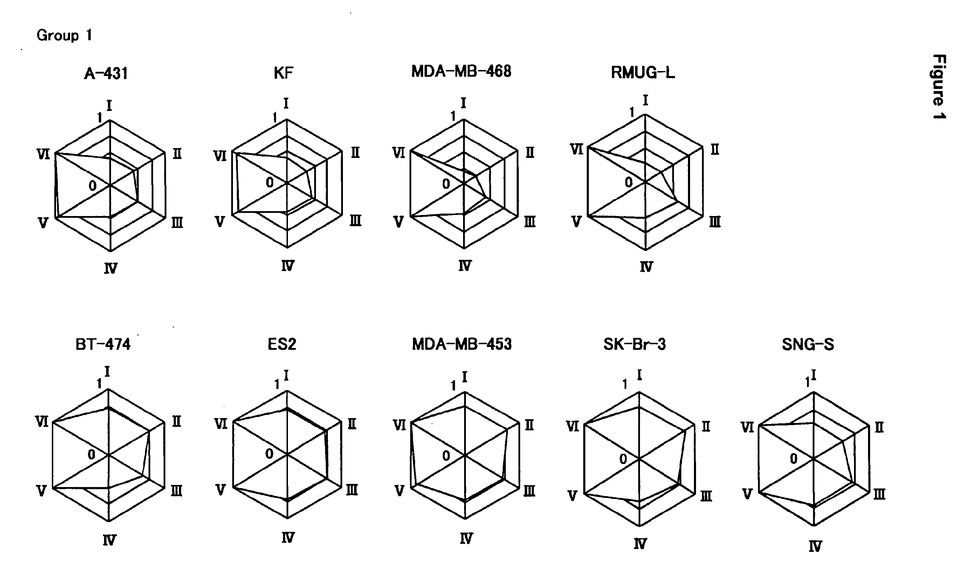

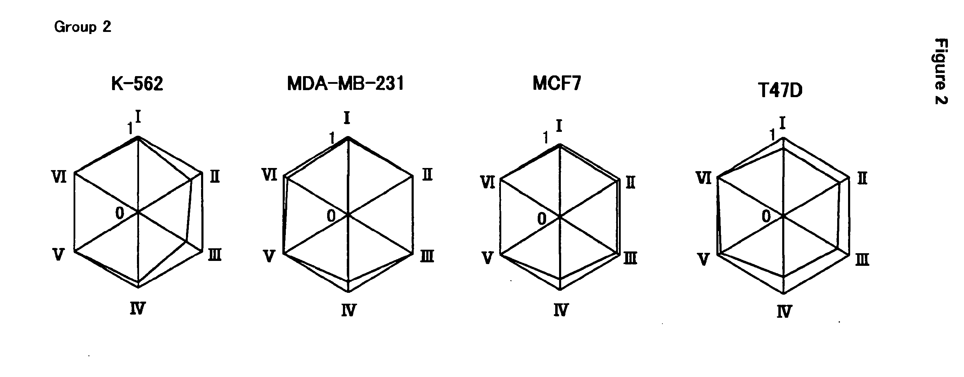

Cobweb charts of Figs. 1 and 2 were generated based on values (relative values) obtained by dividing RTK activities (first RTK activities) obtained with the cell membrane fraction contacted with the inhibitor by RTK activities (control RTK activities) obtained with the cell membrane fraction without a contact with the inhibitor. In Figs. 1 and 2, the relative values of first RTK activities to control RTK activities are shown when I: PD153035, II: AG1478, III: 4557W, IV: Src inhibitor, V: PDGFR inhibitor and VI: VEGFR inhibitor were used as the inhibitors, respectively.

Results of Figs. 1 and 2 show that 13 different tumor cells used in Example 1 can be classified to a group having low relative values for RTK activity in the presence of certain RTK activity inhibitors (A-431, KF, MDA-MB-468, RMUG-L, BT-474, ES2, MDA-MB-453, SK-Br-3 and SNG-S) and a group having relatively high relative values for RTK activity in the presence of all RTK activity inhibitors (K-562, MDA-MB-231, MCF7 and T47D). The group of the cells having low relative values is labeled as group 1 and shown in Fig. 1. The group of the cells having high relative values is labeled as group 2 and shown in Fig. 2.
Results from group 1 in Fig. 1 also show that Src inhibitor used in this Example (inhibitor of non-receptor type tyrosine kinase, Src) inhibits RTK activity.

### Comparative Example: Direct evaluation of the degree of malignancy of tumor cells

According to results of Example 1, tumor cells were able to be classified into groups 1 and 2 based on the first RTK activity and control RTK activity. MDA-MB-468 and BT-474, and MCF7 and MDA-MB-231 were selected from groups 1 and 2, respectively. In order to show that the relative values based on RTK activity obtained in Example 1 correlate with the degree of malignancy of tumor cells in the presence of RTK activity inhibitors, these four cell lines were used for the following experiments.

### Comparative Example 1: Evaluation of proliferative ability of tumor cells

### (Treatment with RTK activity inhibitors and cell culture)

The above four cell lines were respectively seeded into wells of the culture plate (96 Well Solid White Flat Bottom Polystyrene TC-Treated Microplates; Corning) at 1000 cells/well, and cultured in a medium (10% FBS) at 37°C. After 24 hours, the medium was changed to a medium containing 0.078, 0.156, 0.313, 0.625, 1.25 or 2.5µM of PD153035 or AG1478 as RTK activity inhibitor, and the culture was continued at 37°C for additional 3 days.

### (Measurement of viable cells)

Viable cells were measured with CellTiter-Glo Luminescent Cell Viability Assay (Promega). This is a reagent kit which measures viable cells by quantifying ATP originated from cells having metabolic activity in media.
Measurement reagent was prepared according to the protocol attached to the kit. To the wells containing tumor cells cultured in the presence of RTK activity inhibitors as above was added with 100µl/well of the measurement reagent and the plate was agitated on a shaker for two minutes. After agitation, the plate was left to stand for 10 minutes and luminescence was measured on the microplate reader GENios (TECAN). Because luminescence is proportional to the amount of ATP originated from cells having metabolic activity in the medium, the measurement of the luminescence allows the measurement of the number of viable cells in the medium.
Results for 3 wells were obtained for each condition and the average and standard deviation were calculated.
From these results, proliferative ability of tumor cells was evaluated.

### (Results)

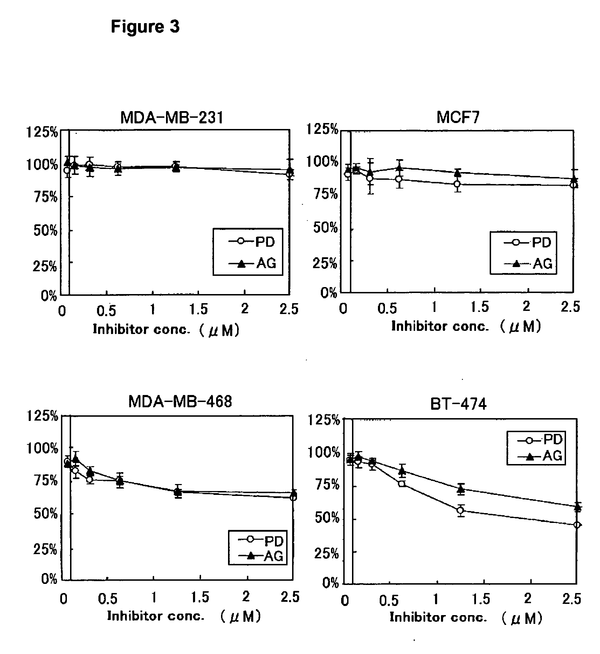

The results of the measurements are shown in Fig. 3. In Fig. 3, the y-axis corresponds to a relative value for the viable cell number in the presence of the inhibitor provided that the viable cell number in the absence of the inhibitor is 100%, and the x-axis corresponds to the inhibitor concentration (µM). In Fig. 3, "PD" and "AG" represent the results of the cells treated with PD153035 and AG1478, respectively.
The results of Fig. 3 show that viable cell number of MCF7 and MDA-MB-231 was not significantly decreased in the presence of RTK activity inhibitors, while that of MDA-MB-468 and BT-474 decreased in the presence of RTK activity inhibitors. Thus, it is shown that proliferative ability of MCF7 and MDA-MB-231 was not significantly decreased in the presence of RTK activity inhibitors, while that of MDA-MB-468 and BT-474 was decreased in the presence of RTK activity inhibitors.
According to the cobweb charts in Fig. 2, RTK activities of MCF7 and MDA-MB-231 were not decreased in the presence of PD153035 or AG1478, while according to the cobweb charts in Fig. 1, RTK activities of MDA-MB-468 and BT-474 were decreased in the presence of PD153035 and AG1478. These results show the same tendency as the results for the proliferative ability of tumor cells in Fig. 3. Thus, it was found that the present method allows the evaluation of proliferative ability of tumor cells in the presence of RTK activity inhibitors.

### Comparative Example 2: Evaluation of size of tumor cells

The following experiments were carried out in order to study the effect of RTK activity inhibitors on proliferative activity of tumor cells (MDA-MB-468) in mice.

### (Tumor formation in mice)

MDA-MB-468 cells were cultured in a medium (DMEM-F12 (Sigma) containing 10% FBS (Hyclone)) until cells become 80% confluent in a 225-cm² flask. The obtained cultured cells were suspended in 100µl of DMEM-F12 at about 1 x 10⁷ cells to obtain a MDA-MB-468 cell suspension.
Fat pad of 10-week-old female mice (BALB/c nu/nu) was injected with 100µl of the MDA-MB-468 cell suspension. After 14 days, the growth of tumor in mice was confirmed. Thus, tumor was allowed to be formed in three mice.

### (Treatment with RTK activity inhibitor)

At 14 days after the injection of the MDA-MB-468 cell suspension, an inhibitor solution was injected to the mouse having tumor. Specifically, AG1478 was dissolved in 100µl of dimethylsulfoxide (DMSO, Sigma) to obtain a inhibitor solution 1. The mouse was injected with the inhibitor solution 1 so that it received the inhibitor at a dose of 30 mg/kg/day. The inhibitor solution 1 was injected for consecutive 7 days. 4557W was dissolved in DMSO to obtain an inhibitor solution 2. Another mouse was injected with the inhibitor solution 2 in the same manner as for the inhibitor solution 1. The third mouse was injected with DMSO only, serving as the negative control.

### (Measurement of tumor mass)

On Day 1, the mice were injected with the inhibitor solutions. On Days 1, 3, 5, and 8, the tumor mass (mm³) in mice was measured. The tumor mass was calculated from the longitudinal and transverse diameters of the tumor measured, assuming that the shape of the tumor is an oval sphere.
The tumor mass of the mouse injected with DMSO was measured in a similar manner.

### (Results)

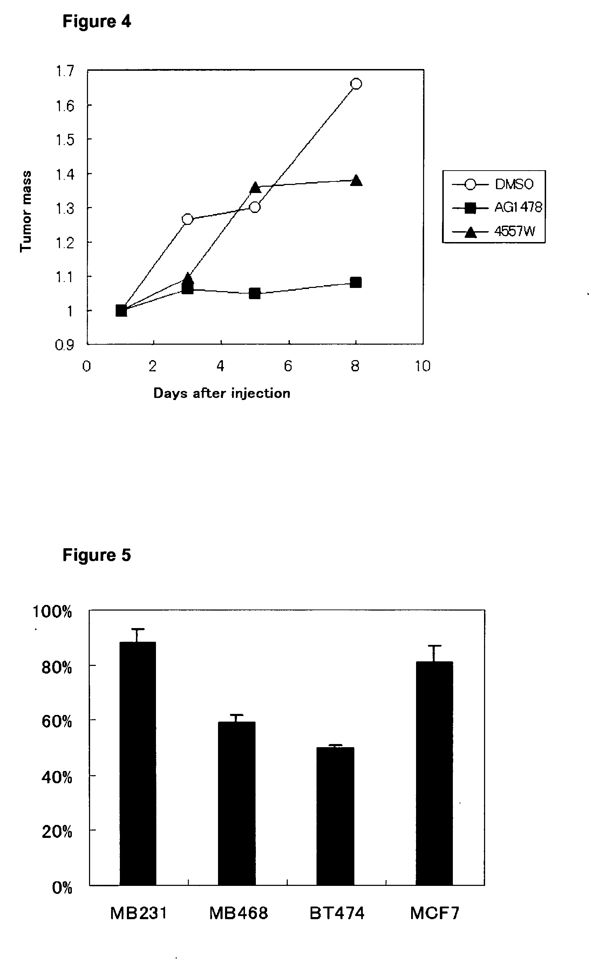

Results are shown in Fig. 4. Fig. 4 shows the change in tumor mass at Days 3, 5 and 8 provided that the mass at Day 1 is 1. In Fig. 4, "DMSO", "AG1478" and "4557W" represent the results of negative control, of the mouse injected with AG1478 and of the mouse injected with 4557W, respectively.
The results in Fig. 4 show that the increase in the tumor mass with MDA-MB-468 cells was suppressed in the presence of AG1478 and 4557W.
According to the cobweb charts in Fig.1, RTK activity of MDA-MB-468 cells was decreased in the presence of AG1478 and 4557W. This shows the same tendency as the results on the suppression of increase in tumor mass in Fig. 4. Thus, it was found that the present method allows the evaluation of the ability of increasing tumor mass, i.e. proliferative ability of tumor cells in the presence of RTK activity inhibitors.

### Comparative Example 3: Evaluation of proliferative ability of tumor cells

Viable cell number was measured as Comparative Example 1 except that the mixture of PD153035, AG1478, 4557W, PDGFR inhibitor and VEGFR inhibitor was used, instead of PD153035 or AG1478, so that each inhibitor used was at 1µM.

### (Results)

Fig. 5 shows the relative values of viable cell number in the presence of the inhibitor provided that the viable cell number in the absence of the inhibitor is 100%.
It is found that, as similar to the results obtained in Comparative Example 1, viable cell number of MCF7 and MDA-MB-231 was not significantly decreased in the presence of RTK activity inhibitors, while that of MDA-MB-468 and BT-474 was decreased in the presence of RTK activity inhibitors. Thus, it is shown that proliferative ability of MCF7 and MDA-MB-231 was not significantly decreased in the presence of RTK activity inhibitors, while that of MDA-MB-468 and BT-474 was decreased in the presence of RTK activity inhibitors.

### Comparative Example 4: Evaluation of secretion ability of vascular endothelial growth factors (VEGFs) of cells

VEGFs are glycoproteins known to be involved in angiogenesis or metastasis of tumors. When VEGFs are secreted from tumor cells, these cells can generate new vessels to obtain further nutrients, propagate, and then be metastasized through generated vessels. Thus, VEGF secretion ability is an index of metastasizing.
The VEGF secretion ability was measured as follows.

Cultured cells of MDA-MB-468, BT-474, MCF7 and MDA-MB-231 were seeded in a 6-well plate (Corning) at 1 x 10⁶ cells/well and cultured at 37°C in a medium (10% FBS). After 24 hours, the medium is changed to a serum free medium and the culture was continued for further 18 hours. The serum free medium used was DMEM/F12 (SIGMA) or RPM11640 (SIGMA) supplemented with the antibiotics Antibiotic-Antimycotic (100x) (GIBCO). Thereafter, the medium was changed to the medium (10% FBS) containing 1µM inhibitor in DMSO (PD153035, AG1478, PDGFR inhibitor or VEGFR inhibitor) and the culture was continued for additional 4 hours. The cell supernatant was then collected. The amount of VEGFs in the supernatant was measured with Human VEGF Quantikine ELISA Kit (R&D system) according to the attached protocol.
The control experiment was carried out with DMSO only instead of the inhibitor.

### (Results)

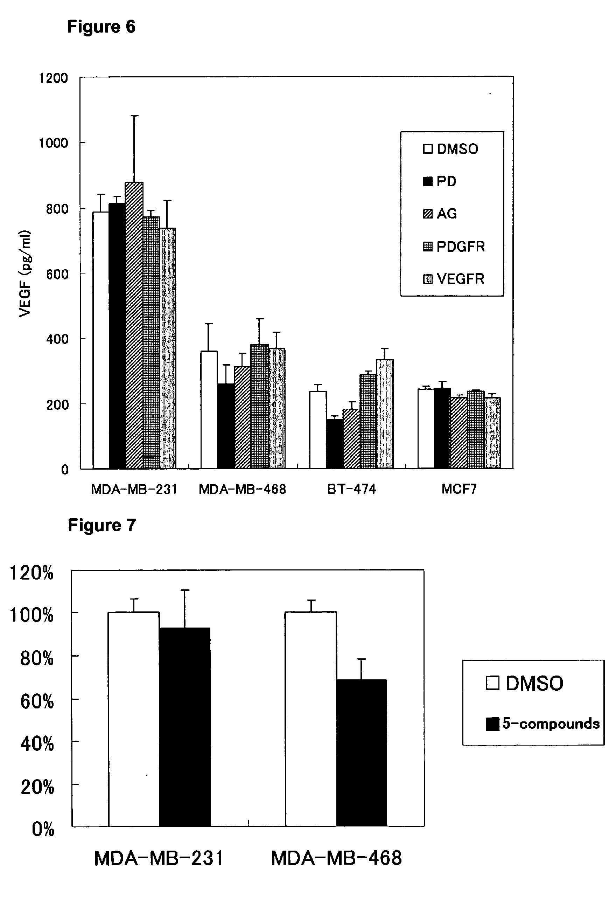

The results are shown in Fig. 6. In Fig. 6, the y-axis corresponds to VEGF concentration (pg/ml), which reflects the amount of VEGFs secreted from the cells. In Fig. 6, "DMSO", "PD", "AG", "PDGFR" and "VEGFR" represent the results of control, and the cells treated with PD153035, AG1478, PDGFR inhibitor and VEGFR inhibitor, respectively.

The results of Fig. 6 show that the VEGF secretion amount from MDA-MB-468 and BT-474 cells was decreased compared to the control value after the treatment with PD153035 or AG1478. Therefore, it is considered that angiogenesis ability (i.e. metastasizing ability) of these cells was decreased in the presence of PD153035 or AG1478.
According to the cobweb charts in Fig. 1, RTK activity of these cells was decreased in the presence of PD153035 and AG1478, respectively. This shows the same tendency as the results of the VEGF secretion amount in Fig. 6. Thus, it was found that the present method allows the evaluation of angiogenesis ability (i.e. metastasizing ability) of tumor cells in the presence of RTK activity inhibitors.

### Comparative Example 5: Evaluation of invasive ability of cells

Tumor cells invade a vessel wall, enter the vessel, are transferred through blood circulation and metastasize to other tissues. Thus, invasive ability of the cells is an index of liability to metastasize, i.e. metastasizing ability of the cells.
Invasive ability of MDA-MB-468 and MDA-MB-231 cells was measured as follows.

The principle of the experiment for measuring invasive ability is as follows. This experiment is carried out with a 96-well chamber having the upper and lower chambers, which is included in Cultrex 96 well Cell Invasion Assay kit (Trevigen). On the bottom of the upper chamber is formed a basement membrane (basement membrane extract; BME), and serum-starved cells are placed on the membrane. The lower chamber is charged with a serum medium. When cells are cultured under this situation, the cells are sought to obtain the nutrition (serum) to move to the lower chamber. By staining the cells moved to the lower chamber with a viable cell-specific fluorescent staining reagent (calcein AM) and measuring its fluorescent intensity, the number of cells moved to the lower chamber can be determined. Thus, the ability for degrading the basement membrane, i.e. invasive ability of the cells can be measured.

### (Treatment with RTK activity inhibitors and cell culture)

The cells were cultured in a medium (10% FBS) at 37°C until cells become 80% confluent in the culture plate Nunclon Surface (Nunc). The medium was then changed to a serum free medium, the culture was continued for 24 hours so that the cells were starved. The serum free medium used was DMEM/F12 (SIGMA) supplemented with the antibiotics Antibiotic-Antimycotic (100x) (GIBCO). The cells were stripped from the wells with 0.05% Trypsin-EDTA (GIBCO), and cells were collected with a quenching buffer (serum free medium containing 5% BSA). The cells were centrifuged to remove Trypsin-EDTA, diluted with the serum free medium so as to obtain the cell suspension of 1 x 10⁶ cells/ml.
The mixture of the inhibitors in DMSO (PD153035, AG1478, 4557W, PDGFR inhibitor and VEGFR inhibitor) was added to the cell suspension so that the concentration of each inhibitor was 1µM.
As the control, only DMSO was added to the cell suspension instead of the inhibitors.

### (Preparation of chambers)

For this experiment, Cultrex 96 well Cell Invasion Assay kit (Trevigen) comprising the 96-well chamber having the upper and lower chambers was used.
According to the attached protocol, respective reagents (0.1 x BME solution, wash buffer, cell dissociation buffer and calcein AM solution) were prepared. To each well of the upper chamber, 50µl of 0.1 x BME solution included in the kit was added, the upper and lower chambers and the lid were assembled and incubated for more than 4 hours at 37°C in a CO₂ incubator. Thus, BME was formed on the bottom of the upper chamber.

### (Experiment for invasive ability)

To each well of the lower chamber was added 150µL of the medium (10% FBS) containing the mixture of the inhibitors in DMSO (PD153035, AG1478, 4557W, PDGFR inhibitor and VEGFR inhibitor) so that the concentration of each inhibitor was 1µM, or the medium containing DMSO was added.
The upper chamber from which the remaining BME solution was removed was assembled with the lower chamber, and 50µl of the cell suspension with or without the inhibitors was added to the respective wells of the upper chamber. When the wells of the lower chamber contain the inhibitor solution, the corresponding wells in the upper chamber also contain the cell suspension containing the inhibitor solution, and vice versa.
The lid was further assembled and the chamber was incubated at 37°C for 40 hours in the CO₂ incubator.

The cell suspension in the upper chamber was removed and wells were washed with 100µL of the wash buffer. The medium in the lower chamber was also removed and wells were washed with 200µL of the wash buffer. A mixture (150µl) obtained by mixing 10 ml of cell dissociation buffer and 12µl of calcein AM solution was added to each well of the lower chamber and the lower chamber was assembled with the upper chamber and lid to be incubated at 37°C for 1 hour. The fluorescence of each well of the lower chamber was measured with the microplate reader GENios (TECAN).

### (Results)

The results obtained are shown in Fig. 7 relative to 100% of the number of the cells moved to the lower chamber when only DMSO was added. In Fig. 7, "MB231 ", "MB468", "DMSO" and "5-compounds" represent MDA-MB-231 cells, MDA-MB-468 cells, control and the case where the inhibitor mixture was added, respectively.
These results show that invasive ability of MDA-MB-231 cells does not significantly alter in the presence of the inhibitors, while that of MDA-MB-468 cells was decreased in the presence of the inhibitors.
According to the cobweb charts in Fig. 2, RTK activity of MDA-MB-231 was not decreased in the presence of the inhibitor, while according to the cobweb charts of Fig. 1, RTK activity of MDA-MB-468 was decreased in the presence of certain inhibitors. This shows the same tendency as the results of invasive ability of tumor cells in Fig. 7. Thus, it was found that the present method allows the evaluation of invasive ability, i.e. metastasizing ability of tumor cells in the presence of RTK activity inhibitors.

### Comparative Example 6: Evaluation of chemotactic ability (migration ability) of cells

Tumor cells can metastasize to other tissues due to the ability of moving, i.e. chemotactic ability. Thus, chemotactic ability of cells is an index of metastasizing ability of the cells.
Chemotactic ability of MDA-MB-468 and MDA-MB-231 cells were measured as follows.

The principle of the experiments for chemotactic ability measurement is as follows. This experiment is also carried out in the same chamber as Comparative Example 5. On the bottom of the upper chamber, pores are formed instead of BME. Serum-starved cells are placed on the bottom of the upper chamber, and the serum medium is placed in the lower chamber. When cells are cultured under this situation, cells are sought to obtain the nutrition (serum) to move to the lower chamber. By staining the cells moved to the lower chamber with a viable cell-specific fluorescent staining reagent (calcein AM) and measuring its fluorescent intensity, the number of cells moved to the lower chamber can be determined. Thus, the ability of moving, i.e. chemotactic ability (migration ability) of cells can be measured.

The same procedures as the measurement of invasive ability in Comparative Example 5 were carried out except that 1 x coating buffer (prepared according to the protocol attached to the kit) was used instead of 0.1 x BME solution during the coating of the bottom of the upper chamber.

### (Results)

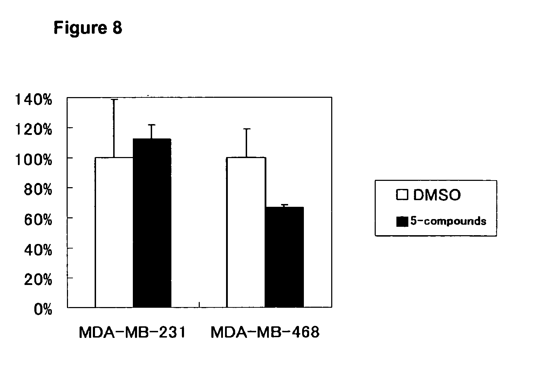

The results obtained are shown in Fig. 8 relative to 100% of the number of the cells moved to the lower chamber when only DMSO was added. In Fig. 8, "MB231 ", "MB468", "DMSO" and "5-compounds" represent MDA-MB-231 cells, MDA-MB-468 cells, control and the case where the inhibitor mixture was added, respectively.
These results show that chemotactic ability of MDA-MB-231 cells does not significantly alter in the presence of the inhibitors, while that of MDA-MB-468 cells was decreased in the presence of the inhibitors.
According to the cobweb charts in Fig. 2, RTK activity of MDA-MB-231 was not decreased in the presence of the inhibitor, while according to the cobweb charts of Fig. 1, RTK activity of MDA-MB-468 decreased in the presence of certain inhibitors. This shows the same tendency as the results of chemotactic ability of tumor cells in Fig. 8. Thus, it was found that the present method allows the evaluation of the chemotactic ability, i.e. metastasizing ability of tumor cells in the presence of RTK activity inhibitors.

According to the above results, it is found that by measuring RTK activity according to the present method as exemplified in Example 1, the degree of malignancy of tumor cells including proliferative ability and metastasizing ability in the presence of RTK activity inhibitors could be evaluated without carrying out complicated experiments such as Comparative Examples 1 to 6.

## Claims

1. A method for evaluating the degree of malignancy of tumor cells comprising the steps of:
(1) preparing a cell membrane fraction containing receptor type tyrosine kinases (RTKs) from a sample containing tumor cells;
(2) bringing the cell membrane fraction obtained in step (1) into contact with a first RTK activity inhibitor;
(3) bringing the cell membrane fraction having been contacted with the RTK activity inhibitor in step (2) into contact with a substrate for at least two RTKs and measuring a phosphorylated substrate, so as to measure RTK activity in the presence of the first RTK activity inhibitor (first RTK activity);
(4) bringing the cell membrane fraction obtained in step (1) into contact with the same substrate as used in step (3) without a contact with the first RTK activity inhibitor and measuring a phosphorylated substrate, so as to measure RTK activity in the absence of the RTK activity inhibitor (control RTK activity);
(5) evaluating the degree of malignancy of the tumor cells in the presence of the first RTK activity inhibitor based on the first RTK activity and the control RTK activity,
wherein step (5) comprises the steps of:
(5a) calculating a relative value for RTK activity of the tumor cells based on the first and control RTK activities; and
(5b) comparing the relative value to a threshold value to evaluate the degree of malignancy of the tumor cells in the presence of the RTK inhibitor,
wherein either:
the relative value is a value obtained by dividing the first RTK activity by the control RTK activity, and if the relative value is lower than the threshold, the degree of malignancy of the tumor cells in the presence of the RTK inhibitor is evaluated to be low, or
the relative value is obtained by subtracting the first RTK activity from the control, RTK activity, and if the relative value is higher than the threshold, the degree of malignancy of the tumor cells in the presence of the RTK inhibitor is evaluated to be low.

2. The method according to claim 1, wherein step (1) comprises the steps of:
(1 a) homogenizing the tumor cells in a buffer;
(1b) obtaining an insoluble fraction from the homogenate obtained in step (1a);
(1c) mixing to the insoluble fraction obtained in step (1 b) with a solubilizing solution containing a surfactant; and
(1d) obtaining a soluble fraction from the mixture obtained in step (1c).

3. The method according to claim 1 or 2, wherein the substrate used in steps (3) and (4) is a substrate mixture in which multiple substrates having high specificities toward certain RTKs are combined, or a substrate having low specificity toward the type of RTKs.

4. The method according to claim 3, wherein the substrate having low specificity toward the type of RTKs comprises a peptide consisting of an amino acid sequence comprising a glutamic acid residue and a tyrosine residue.

5. The method according to claim 1, wherein the RTK activity inhibitor used in step (2) is an adenosine triphosphate (ATP) competitive inhibitor of RTKs.

6. The method according to claim 1, wherein the degree of malignancy of the tumor cells is proliferative ability and/or metastasizing ability of the tumor cells.

7. The method according to claim 1, which further comprises the steps of:
(6) bringing the cell membrane fraction prepared in step (1) into contact with a second RTK activity inhibitor that is different from the first RTK activity inhibitor; and
(7) bringing the cell membrane having contacted with the second RTK activity inhibitor obtained in step (6) into contact with the same substrate as used in step (3) and measuring a phosphorylated substrate, so as to measure RTK activity in the presence of the second RTK activity inhibitor (second RTK activity), and
wherein step (5) further consists in evaluating the degree of malignancy of the tumor cells in the presence of the RTK inhibitors based on the, second and control RTK activities.

8. The method according to claim 7, wherein step (5) further comprises the steps of:
calculating a second relative value for RTK activity of the tumor cells based on the second RTK activity and the control RTK activity; and
comparing the second relative value to a respective corresponding threshold value to further evaluate the degree of malignancy of the tumor cells in the presence of the RTK inhibitors.

## Patentansprüche

1. Verfahren zum Evaluieren des Bösartigkeitsgrades von Tumorzellen, umfassend die folgenden Schritte:
(1) Vorbereiten einer Zellmembranfraktion, welche Rezeptortyp-Tyrosinkinasen (RTKs) aus einer Tumorzellen enthaltenden Probe enthalten;
(2) In-Berührung-bringen der in Schritt (1) erhaltenen Zellmembranfraktion mit einem ersten RTK-Aktivitätsinhibitor;
(3) In-Berührung-bringen der Zellmembran, die in Schritt (2) mit dem RTK-Aktivitätsinhibitor in Berührung gebracht worden ist, mit einem Substrat für mindestens zwei RTKs und Messen eines phosphorylierten Substrates, um RTK-Aktivität in Gegenwart des ersten RTK-Aktivitätsinhibitors zu messen (erste RTK-Aktivität);
(4) In-Berührung-bringen der in Schritt (1) erhaltenen Zellmembranfraktion mit demselben Substrat, wie es in Schritt (3) verwendet wurde, ohne eine Berührung mit dem ersten RTK-Aktivitätsinhibitor, und Messen eines phosphorylierten Substrates, um RTK-Aktivität in Abwesenheit des RTK-Aktivitätsinhibitors zu messen (RTK-Kontrollaktivität);
(5) Evaluieren des Bösartigkeitsgrades der Tumorzellen in Gegenwart des ersten RTK-Aktivitätsinhibitors basierend auf der ersten RTK-Aktivität und der RTK-Kontrollaktivität,
wobei Schritt (5) folgende Schritte umfasst:
(5a) Berechnen eines relativen Wertes für eine RTK-Aktivität der Tumorzellen basierend auf der ersten RTK-Aktivität und der RTK-Kontrollaktivität und
(5b) Vergleichen des relativen Wertes mit einem Schwellenwert, um den Bösartigkeitsgrad der Tumorzellen in Gegenwart des RTK-Aktivitätsinhibitors zu evaluieren,
wobei entweder:
der relative Wert ein Wert ist, welcher durch Dividieren der ersten RTK-Aktivität durch die RTK-Kontrollaktivität erhalten wird, und der Bösartigkeitsgrad der Tumorzellen in Gegenwart des RTK-Aktivitätsinhibitors als niedrig evaluiert wird, wenn der relative Wert niedriger als der Schwellenwert ist, oder
der relative Wert durch Subtrahieren der ersten RTK-Aktivität von der RTK-Kontrollaktivität erhalten wird, und der Bösartigkeitsgrad der Tumorzellen in Gegenwart des RTK-Aktivitätsinhibitors als niedrig evaluiert wird, wenn der relative Wert höher als der Schwellenwert ist.

2. Verfahren nach Anspruch 1, wobei Schritt (1) folgende Schritte umfasst:
(1a) Homogenisieren der Tumorzellen in einem Puffer;
(1b) Erhalten einer unlöslichen Fraktion von dem in Schritt (1a) erhaltenen Homogenat;
(1c) Zumischen einer ein oberflächenaktives Mittel enthaltenden solubilisierenden Lösung zu der in Schritt (1b) erhaltenen unlöslichen Fraktion; und
(1d) Erhalten einer unlöslichen Fraktion aus dem in Schritt (1c) erhaltenen Gemisch.

3. Verfahren nach Anspruch 1 oder 2, wobei es sich bei dem in Schritt (3) und (4) verwendeten Substrat um ein Substratgemisch, in dem mehrere Substrate mit hoher Spezifität für bestimmte RTKs kombiniert sind, oder um ein Substrat mit niedriger Spezifität für den Typ von RTKs handelt.

4. Verfahren nach Anspruch 3, wobei das Substrat mit niedriger Spezifität für den Typ von RTKs ein Peptid umfasst, das aus einer Aminosäuresequenz umfassend einen Glutaminsäurerest und einen Tyrosinrest besteht.

5. Verfahren nach Anspruch 1, wobei es sich bei dem in Schritt (2) verwendeten RTK-Aktivitätsinhibitor um einen Adenosintriphosphat(ATP)-kompetitiven RTK-Inhibitor handelt.

6. Verfahren nach Anspruch 1, wobei es sich bei dem Bösartigkeitsgrad der Tumorzellen um eine proliferative Fähigkeit und/oder Metastasierungsfähigkeit der Tumorzellen handelt.

7. Verfahren nach Anspruch 1, welches des Weiteren folgende Schritte umfasst:
(6) In-Berührung-bringen der in Schritt (1) vorbereiteten Zellmembranfraktion mit einem zweiten RTK-Aktivitätsinhibitor, der sich von dem ersten RTK-Aktivitätsinhibitor unterscheidet, und
(7) In-Berührung-bringen der Zellmembran, die mit dem in Schritt (6) erhaltenen zweiten RTK-Aktivitätsinhibitor in Berührung gebracht worden ist, mit demselben Substrat, das in Schritt (3) verwendet wurde, und Messen eines phosphorylierten Substrates, um RTK-Aktivität in Gegenwart des zweiten RTK-Aktivitätsinhibitors zu messen (zweite RTK-Aktivität), und
wobei Schritt (5) des Weiteren aus dem Evaluieren des Bösartigkeitsgrades der Tumorzellen in Gegenwart der RTK-Inhibitoren basierend auf der zweiten RTK-Aktivität und der RTK-Kontrollaktivität besteht.

8. Verfahren nach Anspruch 7, wobei Schritt (5) des Weiteren folgende Schritte umfasst:
- Berechnen eines zweiten relativen Wertes für eine RTK-Aktivität der Tumorzellen basierend auf der zweiten RTK-Aktivität und der RTK-Kontrollaktivität und
- Vergleichen des zweiten relativen Wertes mit einem entsprechenden Schwellenwert, um des Weiteren den Bösartigkeitsgrad der Tumorzellen in Gegenwart der RTK-Aktivitätsinhibitoren zu evaluieren.

## Revendications

1. Procédé d'évaluation du degré de malignité de cellules tumorales qui comprend les étapes consistant à :
(1) préparer une fraction de membrane cellulaire contenant des tyrosine kinases de type récepteur (RTK) d'un échantillon contenant des cellules tumorales ;
(2) mettre en contact la fraction de membrane cellulaire obtenue dans l'étape (1) avec un premier inhibiteur de l'activité RTK ;
(3) mettre en contact la fraction de membrane cellulaire ayant été mise en contact avec l'inhibiteur de l'activité RTK dans l'étape (2) avec un substrat pour au moins deux RTK et mesurer un substrat phosphorylé, pour mesurer l'activité RTK en présence du premier inhibiteur de l'activité RTK (première activité RTK) ;
(4) mettre en contact la fraction de membrane cellulaire obtenue dans l'étape (1) avec le même substrat que celui utilisé dans l'étape (3) sans contact avec le premier inhibiteur de l'activité RTK et mesurer un substrat phosphorylé, pour mesurer l'activité RTK en l'absence de l'inhibiteur de l'activité RTK (activité RTK témoin) ;
(5) évaluer le degré de malignité des cellules tumorales en présence du premier inhibiteur de l'activité RTK en se basant sur la première activité RTK et sur l'activité RTK témoin,
dans lequel l'étape (5) comprend les étapes consistant à :
(5a) calculer une valeur relative pour l'activité RTK des cellules tumorales en se basant sur la première activité RTK et sur l'activité RTK témoin ; et
(5b) comparer la valeur relative à une valeur seuil pour évaluer le degré de malignité des cellules tumorales en présence de l'inhibiteur des RTK,
dans lequel soit :
la valeur relative est une valeur obtenue par division de la première activité RTK par l'activité RTK témoin, et si la valeur relative est inférieure au seuil, le degré de malignité des cellules tumorales en présence de l'inhibiteur des RTK est évalué comme étant faible, soit
la valeur relative est obtenue par soustraction de la première activité RTK de l'activité RTK témoin, et si la valeur relative est supérieure au seuil, le degré de malignité des cellules tumorales en présence de l'inhibiteur des RTK est évalué comme étant faible.

2. Procédé selon la revendication 1, dans lequel l'étape (1) comprend les étapes consistant à :
(1a) homogénéiser les cellules tumorales dans un tampon,
(1b) obtenir une fraction insoluble de l'homogénat obtenu dans l'étape (1a) ;
(1c) mélanger la fraction insoluble obtenue dans l'étape (1b) avec une solution de solubilisation contenant un surfactant ; et
(1d) obtenir une fraction soluble du mélange obtenu dans l'étape (1c).

3. Procédé selon la revendication 1 ou 2, dans lequel le substrat utilisé dans les étapes (3) et (4) est un mélange de substrats dans lequel de multiples substrats ayant des spécificités élevées envers certaines RTK sont combinés, ou un substrat ayant une faible spécificité envers le type de RTK.

4. Procédé selon la revendication 3, dans lequel le substrat ayant une faible spécificité envers le type de RTK comprend un peptide constitué d'une séquence d'acides aminés comprenant un résidu acide glutamique et un résidu tyrosine.

5. Procédé selon la revendication 1, dans lequel l'inhibiteur de l'activité RTK utilisé dans l'étape (2) est un inhibiteur des RTK compétitif de l'adénosine triphosphate (ATP).

6. Procédé selon la revendication 1, dans lequel le degré de malignité des cellules tumorales est la capacité de prolifération et/ou l'activité de métastase des cellules tumorales.

7. Procédé selon la revendication 1, qui comprend en outre les étapes consistant à :
(6) mettre en contact la fraction de membrane cellulaire préparée dans l'étape (1) avec un second inhibiteur de l'activité RTK qui est différent du premier inhibiteur de l'activité RTK ; et
(7) mettre en contact la membrane cellulaire ayant été en contact avec le second inhibiteur de l'activité RTK obtenue dans l'étape (6) avec le même substrat que celui utilisé dans l'étape (3) et mesurer un substrat phosphorylé, pour mesurer l'activité RTK en présence du second inhibiteur de l'activité RTK (seconde activité RTK), et
dans lequel l'étape (5) consiste en outre à évaluer le degré de malignité des cellules tumorales en présence des inhibiteurs de RTK en se basant sur la seconde activité RTK et sur l'activité RTK témoin.

8. Procédé selon la revendication 7, dans lequel l'étape (5) comprend en outre les étapes consistant à :
- calculer une seconde valeur relative de l'activité RTK des cellules tumorales en se basant sur la seconde activité RTK et sur l'activité RTK témoin ; et
- comparer la seconde valeur relative à une valeur seuil correspondante respective pour ensuite évaluer le degré de malignité des cellules tumorales en présence des inhibiteurs des RTK.
